# EUROPEAN PATENT SPECIFICATION

(11) **EP 4 106 304 B1**
(45) Date of publication and mention of the grant of the patent: **04.09.2024**
(21) Application number: 21753551.7
(22) Date of filing: 20.01.2021
(51) Int. Cl.: H04M 9/02, G16H 40/20

(54) **NURSE CALL SYSTEM**
PFLEGERUFSYSTEM
SYSTÈME D'APPEL D'INFIRMIÈRE

(30) Priority: 10.02.2020 JP 2020020462
(43) Date of publication of application: 21.12.2022
(73) Proprietor: NEC Platforms, Ltd., Kanagawa 213-8511 (JP)
(72) Inventor: NIIYA Hatsue, Kawasaki-shi, Kanagawa 213-8511 (JP)
(74) Representative: Vossius & Partner Patentanwälte Rechtsanwälte mbB
(86) International application number: PCT/JP2021/001752
(87) International publication number: WO 2021/161743

(56) References cited:
- JP-A- 2014 204 751
- JP-A- 2016 087 356
- JP-A- 2018 050 739
- JP-A- 2019 140 428
- JP-A- 2020 000 281
- No further relevant documents disclosed

## Description

### Technical Field

The present disclosure relates to a nurse call system, in particular, to a nurse call system for supporting a plurality of nurses working together to take care of a patient.

### Background Art

In recent years, team nursing in which a plurality of healthcare workers work together to provide nursing care for one patient has been widely adopted. A nurse call system for supporting such team nursing has been proposed. An example of such nurse call system is disclosed in Patent Literature 1.

A nurse call system described in Patent Literature 1 includes:
a nurse call slave unit;
a nurse call master unit;
a mobile terminal carried by a healthcare worker;
a nurse call controller configured to control communications performed among the nurse call slave unit, the nurse call master unit, and the mobile terminal;
a private branch exchange for controlling calls between the nurse call slave unit, the nurse call master unit, and the mobile terminal; and
a generation unit connected to the nurse call controller and the mobile terminal in a communicable manner and configured to generate a chat room where a message can be posted in a chat style based on operation of the mobile terminal by the healthcare worker,
in which the chat room is displayable on each mobile terminal carried by a plurality of healthcare workers belonging to a team in charge of a given patient.

JP 2020 000281 A and JP 2016 087356 A relate to nurse call systems and JP 2018 050739 A discloses a nursing care information processing system using position information.

### Citation List

### Patent Literature

Patent Literature 1: Japanese Unexamined Patent Application Publication No. 2019-140428

### Summary of Invention

### Technical Problem

However, in the nurse call system described in Patent Literature 1, the chat room is created only for in-charge nurses who are pre-set to be in charge of the care of the patient. Therefore, even when the in-charge nurses are at locations distant from the location where the patient is at while a nurse who is not the in-charge nurse is at a location close to the location where the patient is at, the nurse who is not in charge of the patient but is at a location close to the location where the patient is at is not invited to the chat room while the in-charge nurses, who are at locations distant from the location where the patient is at, need to respond to the invitation. In other words, with the nurse call system described in Patent Literature 1, work efficiency of team nursing cannot be sufficiently improved.

### Solution to Problem

A nurse call system according to an example embodiment includes:
a nurse call slave unit provided for patient-use;
a nurse call master unit having the slave unit connected thereto;
a mobile terminal carried by a healthcare worker;
an access point for relaying communications between the mobile terminal and the nurse call main unit;
a private branch exchange having a plurality of the nurse call master units installed in a facility connected thereto and being configured to control communications between the nurse call slave unit and the mobile terminal; and
a chat server configured to generate a chat room to be delivered to the mobile terminal in response to a call originated from the nurse call slave unit, in which
the private branch exchange has first information indicating corresponding relationship between the mobile terminal and the healthcare worker, second information indicating corresponding relationship between the patient and an in-charge healthcare worker who is in charge of the patient among the healthcare workers, and third information indicating location information of the nurse call slave unit and the mobile terminal, and
the chat server generates, based on the first and the third information, a chat room with the in-charge healthcare worker in charge of a subject patient who originated a call using the nurse call slave unit and a nearby healthcare worker assumed to be at a location closer to the subject patient than the in-charge healthcare worker is as users, and provides the chat room to each mobile terminal held by the in-charge healthcare worker and the nearby healthcare worker.

### Advantageous Effects of Invention

Work efficiency of team nursing can be improved by a nurse call system according to an example embodiment.

### Brief Description of Drawings

Fig. 1 is a block diagram of a nurse call system according to a first example embodiment; and
Fig. 2 is a flowchart for describing operation of the nurse call system according to the first example embodiment.

### Example Embodiment

### First Example Embodiment

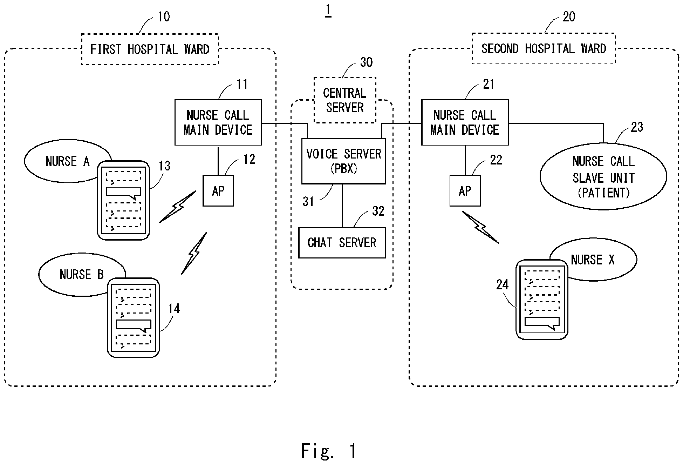

Hereinbelow, an example embodiment of the present disclosure will be described with reference to the drawings. Fig. 1 shows a block diagram of a nurse call system 1 according to the first example embodiment. In the example shown in Fig. 1, the nurse call system 1 is utilized in a facility consisting of two separate buildings, a first hospital ward 10 and a second hospital ward 20. Note that in the actual nurse call system 1, nurse call master units (e.g. nurse call main devices), nurse call slave units, and mobile terminals may be employed by a number greater than those in the example shown in Fig. 1. Further, in the example shown in Fig. 1, a nurse call main device is installed for each hospital ward, but the system may be configured with one nurse call main device installed for shared-use by multiple hospital wards or multiple floors.

In the example shown in Fig. 1, a central server 30 is employed for equipment of shared-use by the first hospital ward 10 and the second hospital ward 20. The central server 30 includes a voice server 31 and a chat server 32. Further, the first hospital ward 10 is equipped with a nurse call main device 11, an access point 12, a mobile terminal 13, and a mobile terminal 14. The second hospital ward 20 is equipped with a nurse call main device 21, an access point 22, a nurse call slave unit 23, and a mobile terminal 24.

The nurse call slave unit 23 is provided for patient-use. To be more specific, the nurse call slave unit 23 is installed so as to be attached to the bed used by the patient. That is, the nurse call slave unit 23 is installed at a fixed location. Further, the location information of the nurse call slave unit 23 is registered in the nurse call main device and the private branch exchange at the time of installation of the nurse call slave unit 23. The nurse call slave unit 23 originates a nurse call, for instance, when a patient presses a button. The nurse call main devices 11 and 21 have the nurse call slave unit 23 and the access points 12 and 22 connected thereto. In the example shown in Fig. 1, the nurse call main device 11 has the access point 12 connected thereto, and the nurse call main device 21 has the nurse call slave unit 23 and the access point 22 connected thereto.

The mobile terminals 13 and 14 and the potable terminal 24 are terminals carried by the healthcare workers. As the mobile terminal, a terminal capable of running a software such as an on-premises PHS (personal handy-phone system), a mobile information terminal, a tablet terminal, and the like can be utilized. The access points 12 and 22 relay communication between the mobile terminals and the aforementioned nurse call main devices. The access points 12 and 22 function as a master unit of the wireless LAN when the terminals that belong to the access points are terminals such as a mobile phone or the like. The access points 12 and 22 function as a radio base station when the terminals that belong to the access points are PHSs or the like. Further, the mobile terminals are connected to the nurse call main device via the access points. In the example shown in Fig. 1, the mobile terminals 13 and 14 are connected to the nurse call main device 11 via the access point 12. Further, the mobile terminal 24 is connected to the nurse call main device 21 via the access point 22. Here, the access points and the mobile terminals perform communication utilizing a radio signal. Note that in the example shown in Fig. 1, the mobile terminal 13 is allocated to Nurse A, the mobile terminal 14 is allocated to Nurse B, and the mobile terminal 24 is allocated to Nurse X. Further, in the nurse call system 1 according to the first example embodiment, it is assumed that the corresponding relationship between the nurses and the mobile terminals allocated to the nurses is held in the voice server 31.

The voice server 31 is, for example, a PBX (Private Branch eXchange). The voice server 31 has the nurse call main devices 11 and 21 connected thereto, the nurse call main devices being installed within the facility by a plural number, and is configured to control communication between the nurse call slave unit 23 and the mobile terminals 13, 14, and 24. In response to a call originated from the nurse call slave unit 23, the chat server 32 generates a chat room to be delivered to the mobile terminals 13, 14, and 24.

Here, in the nurse call system 1 according to the first example embodiment, the voice server 31 has first to third information. The first information is information indicating corresponding relationship between the mobile terminals 13, 14, and 24 and healthcare workers (e.g. Nurse A, Nurse B, and Nurse X). The second information is information indicating corresponding relationship between the patient and in-charge healthcare workers who are in charge of the patient among the nurses (e.g. in-charge nurses). The third information is information indicating location information of the nurse call slave unit 23 and the mobile terminals 13, 14, and 24.

Since the nurse call slave unit 23 is installed at a fixed location, the location information of the nurse call slave unit 23 is registered as the third information at the time of installation of the nurse call slave unit 23. Further, the location information of the mobile terminals includes at least one of the location information of the mobile terminals acquired using a location information acquisition function (e.g. the GPS (Global Positioning System)) built-in in the mobile terminals and the location information of the access points to which the mobile terminals belong. Note that since the access points are installed at fixed locations, the location information of the access points is registered as the third information at the time of installation of the access points.

Further, the chat server 32 generates, based on the first to the third information, a chat room with the in-charge nurses who are in charge of a subject patient who originated a call using the nurse call slave unit 23 and a nearby healthcare worker assumed to be at a location closer to the subject patient than the in-charge nurses are (e.g. a nearby nurse) as users, and provides the chat room to the mobile terminals 13, 14, and 24 held by the in-charge nurses and the nearby nurse. Here, when the location relationship between the mobile terminals and the nurse call slave unit is determined based on the location information of the access points, the chat server 32 simply uses the locations of the access points to which the mobile terminals belong as the locations of the mobile terminals.

Note that in the example shown in Fig. 1, Nurse A and Nurse B are the in-charge nurses who have corresponding relationship with the nurse call slave unit 23, and Nurse X is the nearby nurse. Further, Fig. 1 shows a state in which at the timing when a call is originated from the nurse call slave unit 23, the in-charge Nurses A and B are at a first hospital ward 10 that is different from a second hospital ward 20 where the patient is at, and in the case where the in-charge Nurses A and B cannot take prompt action in response to the call, the nearby Nurse X is requested to take care of the patient.

In the nurse call system 1 according to the first example embodiment, the access point 12 generates a chat room that includes as users the nearby Nurse X in addition to the in-charge Nurses A and B. By this configuration, in the nurse call system 1 according to the first example embodiment, improvement in the operating efficiency of team nursing is realized. Next, operation of the nurse call system 1 according to the first example embodiment will be described hereinbelow.

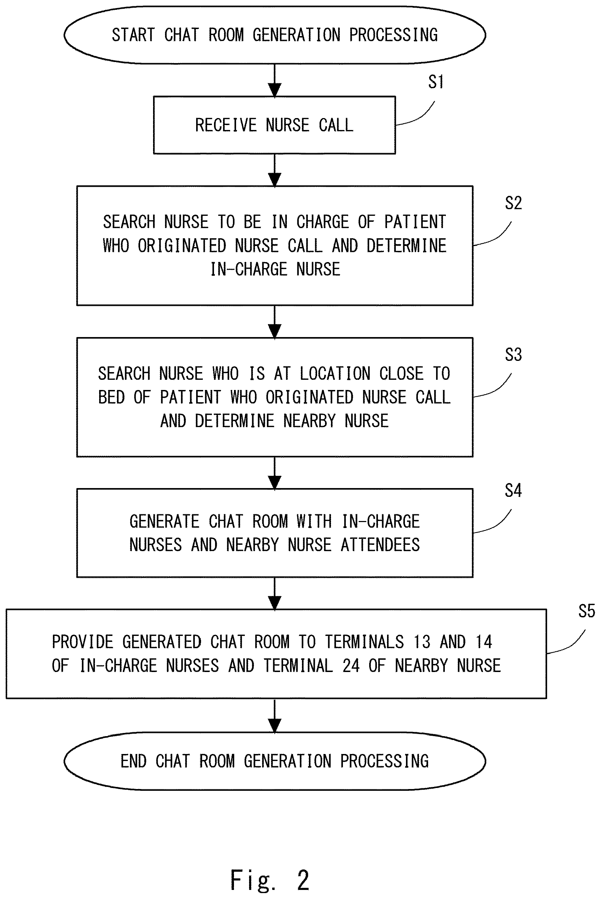

Fig. 2 shows a flowchart describing operation of the nurse call system 1 according to the first example embodiment. As shown in Fig. 2, in the nurse call system 1 according to the first example embodiment, chat room generation processing is started when a call is originated from the nurse call slave unit 23 (Step S1). Then, in the nurse call system 1 according to the first example embodiment, the chat server 32 receives the first to the third information from the voice server 31 and performs processing of Steps S1 to S5. In Step S2, a nurse to be in charge of the patient who originated the nurse call (e.g. the subject patient) is searched from the first and the second information and the in-charge nurse is determined. In Step S3, a nurse who is at a location close to the bed of the subject patient who originated the nurse call is searched and determined as the nearby nurse.

In the nurse call system 1 according to the first example embodiment, the access points are installed at fixed locations, and the voice server 31 generates the third information by estimating each mobile terminal from the location of the access point with which each mobile terminal is performing communication. Further, in the nurse call system 1 according to the first example embodiment, the mobile terminal switch the access point with which it performs communication as the nurse moves. Then, the voice server 31 updates the third information every time the access point with which the mobile terminal performs communication is switched. Further, the third information includes location information of the nurse call slave unit. Further, in the exploring processing of Step S3, based on the aforementioned third information, the nearby nurse who is at a location close to the patient is determined in accordance with the location of the nurse call slave unit from which the nurse call was originated and the mobile terminal which is at a location close to the aforementioned nurse call slave unit. Note that the nearby nurse is a nurse who is at a location closer to the subject patient than the in-charge nurses are. Alternatively, the nearby nurse may select a nurse who is close to the subject patient locationwise.

Next, in Step S4, a chat room with the in-charge nurses and the nearby nurse as attendees is generated. Then, in Step S5, the chat room generated in Step S4 is provided to the mobile terminals 13 and 14 held by the in-charge nurses (e.g. nurses A and B in Fig. 1) and the mobile terminal 24 held by the nearby nurse (e.g. nurse X in Fig. 1).

As described above, in the nurse call system 1 according to the first example embodiment, even when the in-charge nurses are at locations distant from the location where the subject patient is at, they can request, through the chat room, the nearby nurse who is nearby the subject patient to provide care for the subject patient. By this configuration, it is possible to improve the efficiency of team nursing in the nurse call system 1 according to the first example embodiment.

The present disclosure has been described with reference to the example embodiments. However, it should be noted that the present disclosure is to be limited in any way by the example embodiments described above. The configuration and details of the present disclosure can be modified in various ways within the scope of the appended claims.

### Reference Signs List

- 1: NURSE CALL SYSTEM
- 10: FIRST HOSPITAL WARD
- 11: NURSE CALL MAIN DEVICE
- 12: ACCESS POINT
- 13: MOBILE TERMINAL
- 14: MOBILE TERMINAL
- 20: SECOND HOSPITAL WARD
- 21: NURSE CALL MAIN DEVICE
- 22: ACCESS POINT
- 23: NURSE CALL SLAVE UNIT
- 24: MOBILE TERMINAL
- 30: CENTRAL SERVER
- 31: VOICE SERVER
- 32: CHAT SERVER

## Claims

1. A nurse call system (1) comprising:
a nurse call slave unit (23) provided for patient-use;
a nurse call master unit (11, 21) having the nurse call slave unit (23) connected thereto;
a mobile terminal (13, 14, 24) carried by a healthcare worker;
an access point (12,22) for relaying communications between the mobile terminal (13, 14, 24) and the nurse call master unit (11, 21);
a private branch exchange (31) having a plurality of the nurse call master unit (11, 21) installed in a facility connected thereto and being configured to control communications between the nurse call slave unit (23) and the mobile terminal (13, 14, 24); and
a chat server (32) configured to generate a chat room to be delivered to the mobile terminal (13, 14, 24) in response to a call originated from the nurse call slave unit (23), wherein
the private branch exchange (31) has first information indicating corresponding relationship between the mobile terminal (13, 14, 24) and the healthcare worker, second information indicating corresponding relationship between the patient and an in-charge healthcare worker who is in charge of the patient among the healthcare workers, and third information indicating location information of the nurse call slave unit (23) and the mobile terminal (13, 14, 24), and
the chat server (32) is configured to generate, based on the first and the third information, a chat room with the in-charge healthcare worker in charge of a subject patient who originated a call using the nurse call slave unit (23) and a nearby healthcare worker assumed to be at a location closer to the subject patient than the in-charge healthcare worker is as users, and to provide the chat room to each mobile terminal (13, 14, 24) held by the in-charge healthcare worker and the nearby healthcare worker.

2. The nurse call system (1) according to Claim 1, wherein the private branch exchange (31) is configured to update the third information based on at least one of location information of the access point (12,22) with which the mobile terminal (13, 14, 24) performs communication and location information acquired by the mobile terminal (13, 14, 24).

3. The nurse call system (1) according to Claim 2, wherein when location relationship between the mobile terminal (13, 14, 24) and the nurse call slave unit (23) is determined based on the location information of the access point (12,22), the chat server (32) is configured to simply use the location information of the access point (12,22) to which the mobile terminal (13, 14, 24) belongs as the location of the mobile terminal (13, 14, 24).

4. The nurse call system (1) according to Claim 2, wherein the mobile terminal (13, 14, 24) is configured to acquire the location information based on the Global Positioning System and to transmit the location information acquired in response to a request from the private branch exchange (31) to the private branch exchange (31).

5. The nurse call system (1) according to any one of Claims 1 to 4, wherein the chat server (32) and the private branch exchange (31) are built-in in one device.

6. The nurse call system (1) according to any one of Claims 1 to 5, wherein the access point (12,22) is provided for each facility where the health care workers work, each floor of the facility, or each work area on the same floor of the facility.

7. The nurse call system (1) according to any one of Claims 1 to 6, wherein the chat server (32) is configured to select a healthcare worker who is at a location closest to the subject patient as the nearby healthcare worker.

8. A nurse call method for a nurse call system including a nurse call slave unit (23) provided for patient-use and a mobile terminal (13, 14, 24) carried by a healthcare worker, the method comprising:
generating, based on first to third information, a chat room with an in-charge healthcare worker in charge of a subject patient who originated a call using the nurse call slave unit (23) and a nearby healthcare worker assumed to be at a location closer to the subject patient than the in-charge healthcare worker is as users,
the first information indicating corresponding relationship between the mobile terminal (13, 14, 24) and the healthcare worker,
the second information indicating corresponding relationship between the patient and an in-charge healthcare worker who is in charge of the patient among the healthcare workers, and
the third information indicating location information of the nurse call slave unit (23) and the mobile terminal (13, 14, 24); and
providing the chat room to each mobile terminal (13, 14, 24) held by the in-charge healthcare worker and the nearby healthcare worker.

9. The nurse call method according to Claim 8, wherein a healthcare worker who is at a location closest to the subject patient is selected as the nearby healthcare worker.

10. A program for causing a computer to execute the nurse call method according to Claim 8 or 9.

## Patentansprüche

1. Schwesternrufsystem (1), das Folgendes aufweist:
eine Schwestemruf-Nebeneinheit (23), die zur Patientennutzung vorgesehen ist;
eine Schwesternruf-Haupteinheit (11, 21), mit der die Schwesternruf-Nebeneinheit (23) verbunden ist;
ein mobiles Endgerät (13, 14, 24), das von einem medizinischen Mitarbeiter getragen wird;
einen Zugangspunkt (12, 22) zur Weiterleitung der Kommunikation zwischen dem mobilen Endgerät (13, 14, 24) und der Schwesternruf-Haupteinheit (11, 21);
eine Nebenstellenanlage (31) mit mehreren Schwesternruf-Haupteinheiten (11, 21), die in einer damit verbundenen Einrichtung installiert und konfiguriert sind, die Kommunikation zwischen der Schwesternruf-Nebeneinheit (23) und dem mobilen Endgerät (13, 14, 24) zu steuern; und
einen Chat-Server (32), der konfiguriert ist, einen Chat-Raum zu erzeugen, der als Reaktion auf einen von der Schwesternruf-Nebeneinheit (23) ausgehenden Anruf an das mobile Endgerät (13, 14, 24) übermittelt wird, wobei
die Nebenstellenanlage (31) erste Informationen, die eine entsprechende Beziehung zwischen dem mobilen Endgerät (13, 14, 24) und dem medizinischen Mitarbeiter anzeigen, zweite Informationen, die eine entsprechende Beziehung zwischen dem Patienten und einem zuständigen medizinischen Mitarbeiter anzeigen, der für den Patienten unter den medizinischen Mitarbeitern zuständig ist, und dritte Informationen aufweist, die eine Standortinformation der Schwesternruf-Nebeneinheit (23) und des mobilen Endgeräts (13, 14, 24) anzeigen, und
der Chat-Server (32) konfiguriert ist, basierend auf den ersten und der dritten Informationen einen Chat-Raum mit dem zuständigen medizinischen Mitarbeiter, der für einen Patienten zuständig ist, der einen Anruf unter Verwendung der Schwesternruf-Nebeneinheit (23) getätigt hat, und einem medizinischen Mitarbeiter in der Nähe, von dem angenommen wird, dass er sich an einem Ort befindet, der sich näher am Patienten befindet als der zuständige medizinische Mitarbeiter, als Benutzer zu erzeugen und den Chat-Raum jedem mobilen Endgerät (13, 14, 24) bereitzustellen, das vom zuständigen medizinischen Mitarbeiter und dem medizinischen Mitarbeiter in der Nähe getragen wird.

2. Schwesternrufsystem (1) nach Anspruch 1, wobei die Nebenstellenanlage (31) konfiguriert ist, die dritten Informationen basierend auf Standortinformationen des Zugangspunkts (12, 22), mit dem das mobile Endgerät (13, 14, 24) eine Kommunikation durchführt, und/oder vom mobilen Endgerät (13, 14, 24) erfassten Standortinformationen zu aktualisieren.

3. Schwesternrufsystem (1) nach Anspruch 2, wobei, wenn die Standortbeziehung zwischen dem mobilen Endgerät (13, 14, 24) und der Schwesternruf-Nebeneinheit (23) basierend auf den Standortinformationen des Zugangspunkts (12, 22) bestimmt wird, der Chat-Server (32) konfiguriert ist, einfach die Standortinformationen des Zugangspunkts (12, 22), zu dem das mobile Endgerät (13, 14, 24) gehört, als den Standort des mobilen Endgeräts (13, 14, 24) zu verwenden.

4. Schwesternrufsystem (1) nach Anspruch 2, wobei das mobile Endgerät (13, 14, 24) konfiguriert ist, die Standortinformationen basierend auf dem globalen Positionsbestimmungssystem zu erfassen und die erfassten Standortinformationen als Reaktion auf eine Anfrage von der Nebenstellenanlage (31) an die Nebenstellenanlage (31) zu übermitteln.

5. Schwesternrufsystem (1) nach einem der Ansprüche 1 bis 4, wobei der Chat-Server (32) und die Nebenstellenanlage (31) in einem Gerät eingebaut sind.

6. Schwesternrufsystem (1) nach einem der Ansprüche 1 bis 5, wobei der Zugangspunkt (12, 22) für jede Einrichtung, in der die medizinischen Mitarbeiter arbeiten, für jedes Stockwerk der Einrichtung oder für jeden Arbeitsbereich auf demselben Stockwerk der Einrichtung vorgesehen ist.

7. Schwesternrufsystem (1) nach einem der Ansprüche 1 bis 6, wobei der Chat-Server (32) konfiguriert ist, einen medizinischen Mitarbeiter, der sich an einem Ort befindet, der dem betreffenden Patienten am nächsten ist, als den in der Nähe befindlichen medizinischen Mitarbeiter auszuwählen.

8. Schwesternrufverfahren für ein Schwesternrufsystem, das eine Schwesternruf-Nebeneinheit (23), die zur Patientennutzung vorgesehen ist, und ein mobiles Endgerät (13, 14, 24) umfasst, das von einem medizinischen Mitarbeiter getragen wird, wobei das Verfahren aufweist:
Erzeugen, basierend auf ersten bis dritten Informationen, eines Chat-Raums mit einem zuständigen medizinischen Mitarbeiter, der für einen betroffenen Patienten zuständig ist, der einen Anruf unter Verwendung der Schwesternruf-Nebeneinheit (23) getätigt hat, und einem medizinischen Mitarbeiter in der Nähe, von dem angenommen wird, dass er sich an einem Ort befindet, der sich näher am betroffenen Patienten als der zuständige medizinischen Mitarbeiter befindet, als Benutzer,
wobei die ersten Informationen eine entsprechende Beziehung zwischen dem mobilen Endgerät (13, 14, 24) und dem medizinischen Mitarbeiter anzeigen,
wobei die zweiten Informationen eine entsprechende Beziehung zwischen dem Patienten und einem zuständigen medizinischen Mitarbeiter anzeigen, der unter den medizinischen Mitarbeitern für den Patienten zuständig ist, und
wobei die dritten Informationen Standortinformationen der Schwesternruf-Nebeneinheit (23) und des mobilen Endgeräts (13, 14, 24) anzeigen; und
Bereitstellen des Chat-Raums für jedes mobile Endgerät (13, 14, 24), das vom zuständigen medizinischen Mitarbeiter und dem in der Nähe befindlichen medizinischen Mitarbeiter getragen wird.

9. Schwesternrufverfahren nach Anspruch 8, wobei ein medizinischer Mitarbeiter, der sich an einem Ort befindet, der dem betroffenen Patienten am nächsten ist, als der in der Nähe befindliche medizinischen Mitarbeiter ausgewählt wird.

10. Programm, um einen Computer zu veranlassen, das Schwesternrufverfahren nach Anspruch 8 oder 9 auszuführen.

## Revendications

1. Système d'appel de personnel infirmier (1), comprenant :
une unité esclave d'appel de personnel infirmier (23) destinée à être utilisée par un patient
une unité maître d'appel de personnel infirmier (11, 21) à laquelle est reliée l'unité esclave d'appel de personnel infirmier (23) ;
un terminal mobile (13, 14, 24) porté par un personnel soignant ;
un point d'accès (12,22) pour relayer les communications entre le terminal mobile (13, 14, 24) et l'unité maître d'appel de personnel infirmier (11, 21) ;
un autocommutateur privé (31) comportant une pluralité d'unités maîtres d'appel de personnel infirmier (11, 21) installées dans un établissement relié à celui-ci, et configuré pour commander les communications entre l'unité esclave d'appel de personnel infirmier (23) et le terminal mobile (13, 14, 24) ; et
un serveur de chat (32) configuré pour générer un salon de discussion à fournir au terminal mobile (13, 14, 24) en réponse à un appel provenant de l'unité esclave d'appel de personnel infirmier (23), où
l'autocommutateur privé (31) dispose de premières informations indiquant une relation de correspondance entre le terminal mobile (13, 14, 24) et le personnel soignant, de deuxièmes informations indiquant la relation de correspondance entre le patient et un personnel soignant en charge du patient parmi les personnels soignants, et de troisièmes informations indiquant les informations de localisation de l'unité esclave d'appel de personnel infirmier (23) et du terminal mobile (13, 14, 24), et où
le serveur de chat (32) est configuré pour générer, sur la base des premières et des troisièmes informations, un salon de discussion avec le personnel soignant en charge d'un patient donné à l'origine d'un appel au moyen de l'unité esclave d'appel de personnel infirmier (23) et un personnel soignant à proximité, supposé se trouver à un endroit plus proche du patient donné que le personnel soignant en charge en tant qu'utilisateurs, et pour fournir le salon de discussion à chaque terminal mobile (13, 14, 24) détenu par le personnel soignant en charge et par le personnel soignant à proximité.

2. Système d'appel de personnel infirmier (1) selon la revendication 1, où l'autocommutateur privé (31) est configuré pour mettre à jour les troisièmes informations sur la base des informations de localisation du point d'accès (12, 22) avec lequel le terminal mobile (13, 14, 24) établit une communication et/ou des informations de localisation acquises par le terminal mobile (13, 14, 24).

3. Système d'appel de personnel infirmier (1) selon la revendication 2, où, lorsque la relation de localisation entre le terminal mobile (13, 14, 24) et l'unité esclave d'appel de personnel infirmier (23) est déterminée sur la base des informations de localisation du point d'accès (12, 22), le serveur de chat (32) est configuré pour utiliser simplement les informations de localisation du point d'accès (12, 22) auquel appartient le terminal mobile (13, 14, 24) comme localisation du terminal mobile (13, 14, 24).

4. Système d'appel de personnel infirmier (1) selon la revendication 2, où le terminal mobile (13, 14, 24) est configuré pour acquérir les informations de localisation sur la base du système de positionnement global et pour transmettre les informations de localisation acquises en réponse à une demande de l'autocommutateur privé (31) à l'autocommutateur privé (31).

5. Système d'appel de personnel infirmier (1) selon l'une des revendications 1 à 4, où le serveur de chat (32) et l'autocommutateur privé (31) sont intégrés dans un seul dispositif.

6. Système d'appel de personnel infirmier (1) selon l'une des revendications 1 à 5, où le point d'accès (12,22) est prévu pour chaque établissement où travaillent les personnels soignants, chaque étage de l'établissement ou chaque zone de travail dans le même étage de l'établissement.

7. Système d'appel de personnel infirmier (1) selon l'une des revendications 1 à 6, où le serveur de chat (32) est configuré pour sélectionner un personnel soignant se trouvant à l' emplacement le plus proche du patient donné en tant que personnel soignant à proximité.

8. Procédé d'appel de personnel infirmier pour un système d'appel de personnel infirmier comprenant une unité esclave d'appel de personnel infirmier (23) destinée à être utilisée par un patient et un terminal mobile (13, 14, 24) porté par un personnel soignant, ledit procédé comprenant :
la génération, sur la base des première aux troisièmes informations, d'un salon de discussion avec un personnel soignant en charge d'un patient à l'origine d'un appel au moyen de l'unité esclave d'appel de personnel infirmier (23) et un personnel soignant à proximité, supposé se trouver à un endroit plus proche du patient donné que le personnel soignant en charge en tant qu'utilisateurs,
les premières informations indiquant une relation de correspondance entre le terminal mobile (13, 14, 24) et le personnel soignant,
les deuxièmes informations indiquant une relation de correspondance entre le patient et un personnel soignant en charge du patient parmi les personnels soignants, et
les troisièmes informations indiquant des informations de localisation de l'unité esclave d'appel de personnel infirmier (23) et du terminal mobile (13, 14, 24) ; et
la fourniture de l'espace de discussion à chaque terminal mobile (13, 14, 24) détenu par le personnel soignant en charge et le personnel soignant à proximité.

9. Procédé d'appel de personnel infirmier selon la revendication 8, où un personnel soignant se trouvant à l'emplacement le plus proche du patient donné est sélectionné en tant que personnel soignant à proximité.

10. Programme, entraînant l'exécution par un ordinateur du procédé d'appel de personnel infirmier selon la revendication 8 ou la revendication 9.
